(19) Europäisches Patentamt
European Patent Office
Office européen des brevets

(11) Publication number: 0 265 508 B1

(12) EUROPEAN PATENT SPECIFICATION

(45) Date of publication of patent specification: 26.08.92

(51) Int. Cl.[5]: C07D 493/04, A61K 31/365

(21) Application number: 87903499.9

(22) Date of filing: 15.04.87

(86) International application number:
PCT/US87/00877

(87) International publication number:
WO 87/06585 (05.11.87 87/24)

(54) **PRODUCTION AND USE OF REACTION PRODUCT OBTAINED FROM ASCORBIC ACID WITH ALPHA,BETA-UNSATURATED ALICYCLIC KETONE OR VINYL ALIPHATIC KETONE.**

(30) Priority: 29.04.86 US 857291

(43) Date of publication of application:
04.05.88 Bulletin 88/18

(45) Publication of the grant of the patent:
26.08.92 Bulletin 92/35

(84) Designated Contracting States:
AT BE CH DE FR GB IT LI LU NL SE

(56) References cited:
US-A- 4 238 500
US-A- 4 287 205

CANADIAN JOURNAL OF CHEMISTRY, vol. 65, 1987, pages 131-136, Ottawa, CA; ARNOLD R. et al.: "The Michael adduct of L-ascorbic acid to methyl vinyl ketone: its remarkable self-condensation and other reactions"

TETRAHEDRON, vol. 39, no. 13, June 1983; FODOR G. et al.: "A new role for L-ascorbic acid: Michael donor to alpha, beta-unsaturated carbonyl compounds", pages 2137-2145

(73) Proprietor: THERACEL CORPORATION
7658 Standish Place, Suite 107
Rockville Maryland 20855(US)

(72) Inventor: FODOR, Gabor, B.
829 Augusta Avenue
Morgantown, WV 26505(US)
Inventor: VELTRI, Robert, W.
19005 Glendower Road
Gaithersburg, MD 20879(US)

(74) Representative: Weinhold, Peter, Dr. et al
Patentanwälte Dr. V. Schmied-Kowarzik
Dipl.-Ing. G. Dannenberg Dr. P. Weinhold Dr.
D. Gudel Dipl.-Ing. S. Schubert Dr. P. Barz
Siegfriedstrasse 8
W-8000 München 40(DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

BACKGROUND OF THE INVENTION

This application is concerned with Michael addition products of selected unsaturated aldehydes and ketones with ascorbic acid. It includes both L-ascorbic acid and its isomer D-ascorbic acid. L-ascorbic acid is also known as vitamin C. It is concerned also with pharmaceutical compositions containing the products as the principal active ingredients, and with methods of using the products to stimulate the immune response in animals.

The compounds of this invention are useful as immunomodulating agents. They can be formulated with conventional pharmaceutical carriers for administration to animals and humans. The compounds and compositions containing them show immunomodulatory activity, especially immunostimulation at very low concentrations. As such they are useful for treatment of a wide variety of mammalian disorders which require stimulation of the immune system. These include, for example, stimulation of the immune system following chemotherapy or radiation therapy. The products are also useful to stimulate the proliferation of helper cells in diseases such as measles, retroviruses (HLTV-III), and leprosy which are characterized by an undesirably high concentration of suppressor cells. They are also useful in the early stages of various infections to stimulate the production of interleukins, interferons, and other natural lymphokines.

The immune system is one of the primary defenses against disease causing microbes and other foreign proteins in higher animals. An immune response is mediated by the action of specific antibody proteins which react to specific antigens. Antigens are substances of fairly high molecular weight, often proteins, which are foreign to an individual's body. They are most frequently located on the outer surfaces of cells. Potential antigens can be found, for example, on pollen grains, tissue grafts. some tumor cell surfaces, animal parasites, viruses, and bacteria.

In humans, many potential antigens never pass the body's first two defense lines and therefore may not provide sufficient stimulation to the immune system. These two primary defense lines consist firstly of the skin, mucous membranes, tears, and stomach acid and secondly of specialized white blood cells, granulocytes and monocytes, and macrophages which may destroy pathogens and other potential antigens by phagocytosis, that is by engulfing and destroying the foreign material. These white blood cells and macrophages are called phagocytes. When pathogens or other foreign substances do pass the body's first two defense lines, the immune response begins.

There are two principal compartments of the immune defense system, humoral and cellular, both of which react to antigens. Humoral immunity is due to circulating antibodies which are found in the gamma globulin fraction of the plasma proteins. When plasma is centrifuged at high speed or chemically precipitated with ethanol by the Cohn procedure its component proteins separate by weight or charge into sections called fractions. Antibodies are usually found in the gamma globulin fraction whose components have a sedimentation constant of about 7-10S and the IgG fraction has a molecular weight of approximately 156,000. Humoral immunity provides long term protection against bacterial and viral infections. Cellular immunity is partly due to direct lymphocyte interaction, or reactions with their products called lymphokines. This type of immunity is responsible for delayed allergic reactions, rejection of transplant of foreign tissue, and rejection of tumor cells. It is the major defense against infections due to viruses, fungi, parasites, and a few bacteria such as the tubercle bacillus and plays a key role in recovery from such infections.

Specialized white blood cells called lymphocytes are responsible for both humoral and cellular immunity. The lymphocyte precursors originate as hematopoietic tissue ontogenetically (pre-natally) in the embryo before the appearance of bone. It is first evident in the yolk sac as "blood islands", small clusters of hematopoietic cells linked with the yolk blood vessels. These islands contain the multipotential hematopoietic cells termed stem cells. As the embryo develops, hemopoietic cells invaginate into the body stock and into the mesenchymal bed in the anterior ventral portion of the abdomen contigous with the stalk. The liver migrates into this same site of the body mesenchyme as an evagination from the gut epithelium, proliferates, and assumes the architecture of hepatic cords among hemopoietic cells. The liver thereby becomes a hemopoietic organ until close to parturition. About half way through gestation the bone cavities begin to demonstrate definite hematopoietic tissue. As mammals approach embryonic maturity hematopoiesis recedes in the liver and the bone marrow becomes the dominant hematopoietic organ.

Post-natally the lymphoid organs of the body house the immunologically competent lymphocytes which characterize the immune system. The bone marrow houses the stem cells (precursor of all myeloid and lymphoid cellular elements). Some of these stem cells migrate to one of the primary lymphoid organs of man and other mammals, the thymus. The thymus is a multilobed organ that lies high behind the sternum. Here, the stem cells proliferate and differentiate into mature T-lymphocytes which then enter the circulation

and seed secondary lymphoid organs including the spleen, lymph nodes, tonsils, appendix, and Peyer's patches in the gut. The bone marrow also seeds the gut-associated lymphoid system, distributed along the gut, with pre-B cells. These cells then proliferate and differentiate under the influence of antigenic stimulation and migrate to the same secondary lymphoid organs described above. The T-cells and B-cells are structurally and functionally distinguishable through various biological, immunochemical and biochemical means.

Humoral immunity is mediated by the B-lymphocytes which have immunoglobulin receptors for particular antigens on their cell surfaces. They seem to be very specific and each type of B-lymphocyte reacts to only one antigen. When bacteria or viruses, for example, invade an organism, B-lymphocytes react to and combine with the antigens on the bacterial or viral surface and the lymphocyte is stimulated to divide. Its daughter cells differentiate into specialized cells called plasma cells. These cells produce and then secrete large quantities of antibodies into the general circulation. The antibodies are specific for the antigens which stimulated their production and react only with those antigens. Antibodies formed in response to antigens by the plasma cells may be functionally differentiated as cytophilic, that is they are capable of combining with cellular antigens and enhancing phagocytosis by monocytes, macrophages and polymorphonuclear granulocytes in the peripheral circulation. Such antibodies may also be cytotoxic and on combination with cellular antigens in the presence of complement may cause lysis. Other antibodies may in the presence of specific antigen-sensitized T-cells produce antibody dependent cell lysis of tumor cells or virus infected cells. Antibodies produced to toxins or viruses may neutralize their toxicity or infectivity respectively by combining with the appropriate critical site for biological activity. Still other antibodies may be directed against the idiotypic determinant of an antibody molecule (the variable domain of the molecule), thereby being defined as an anti-idiotype or anti-antibodies (antibody 2) which are capable of regulating specific antibody synthesis or maintenance of antibody levels. In the latter cascade, antibody may be formed to the anti-idiotype generating a new antibody (antibody 3) with a specificity to the original antigen. The latter may be achieved without the immunized animal ever having experienced challenge with the original antigen. Such technology may be of value in modifying the course of autoimmune or malignant diseases.

Once a pathogen invades the body and the immune response begins, antibodies are made between 10-14 days later. This initial reaction is called the primary response or primary immunization. However, during that time, the pathogens have also been dividing and producing various disease symptoms. It may take days or weeks before enough antibodies are made to eliminate all the pathogens but once they disappear, the disease symptoms disappear as well. The lymphocytes, plasma cells, and antibodies remain and circulate in the blood so that if the same pathogens enter the body a second time, the B-memory lymphocytes react immediately and start antibody production. The response of these pre-sensitized lymphocytes is called the secondary response. The secondary response results in the production of higher levels of antibody than were currently circulating in the plasma. So many antibodies are produced so rapidly that the microbes are unable to establish themselves, divide, and cause disease under the latter circumstances.

Humoral immunity produced by the IgE isotype of immunoglobulin has as one of its efferent reactions immediate hypersensitivity due to the fact that a previously exposed organism can respond within minutes to an antigen, as in the case of hay fever. Another example of immediate hypersensitivity would be anaphylactic shock, an extreme allergic reaction that sometimes occurs when an individual is exposed to an antigen to which he has been sensitized. Sometimes, this humoral response to the antigen can result in death.

Humoral immunity can also be both naturally and artificially induced. In the case of active natural acquired immunity, an individual's B-lymphocytes continue to circulate and activate the production of antibodies after an infection. This active natural acquired immunity lasts for many years or even a lifetime. An infant receives antibodies from the colostrum, milk secreted by the mother, the first few days after birth, which provides immunity during the first year of its life. This is known as passive natural immunity since the infant is not involved in the actual production of the antibodies. Active artificial immunity is induced by injecting dead or weakened (attenuated) microbes or synthetic antigens into an individual. These antigens can still trigger B-lymphocytes to produce antibodies against the causative pathogen. When the individual is later exposed to the virulent microbe, he is already sensitized and immediately responds with a massive secondary (memory) production of antibodies. Active artificial immunity may last many years or permanently with booster shots. There is also a form of passive artificial immunity which provides protection for about one month. This temporary immunity is brought about by injecting antibodies obtained from another person or animal into an individual. It is usually only used in crisis situations and epidemics. Because the lymphocytes are by passed, they neither make antibodies nor "remember" the antigen, which accounts for

the temporary effect of this method.

In cellular immunity, as contrasted to humoral immunity, circulating antibodies are not detectable. The T-lymphocytes which mediate this type of immunity are activated when they encounter antigens on cells from another individual, as in the case of transplants, tumors, bacterial, or parasites or viruses. Like B-lymphocytes, T-lymphocytes are specific and each type reacts with only one antigen. The T-lymphocytes in the peripheral circulation are divided into subpopulations with different effector functions in the immune response. The T-helper inducer subpopulation has a specific receptor for antigen and is responsible for augmentation of the production of specific antibodies to the antigen by B-cells. The T-helper inducer is identified in humans by a surface marker referred to as the T-4 antigen and can be detected with monoclonal antibodies. Another key T-lymphocuted subpopulation is the T-suppressor inducer (T-8 antigen surface marker) lymphocyte which regulates the magnitude of response of certain T- and B-cells to specific antigens. There are also T-cytotoxic (killer) cells which can bind directly to target tumor or graft or virus infected cells causing their destruction. In addition when T-cells proliferate in response to antigen they produce lymphokines which participate in regulation of the immune response as well as removal of the foreign antigen. T-cells are directly involved in cell mediated immunity to tumor cells, virus-infected cells and other cellular antigens and clearly help in recovery from such disease processes. Also, the T-cells are responsible for allograft rejection, delayed, cutaneous hypersensitivity (DCH), chemical sensitization to poison ivy, oak, sumac as well as certain metals. This DCH reaction is called such because it takes 24-48 hours to develop subsequent to exposure to the antigen. Cellular immunity to new antigens usually occurs a few days before the primary (IgM) antibody response occurs in mammals and their are memory T-cells which are responsible for long term immunity. Another T-lymphocyte subpopulation is the natural killer (NK) T-cells (large granular lymphocytes) and these cells are called into action without prior antigenic provocation. These NK cells are active against tumors or virus infected cells and they can be stimulated to higher levels of activity (proliferation) by interferon. These cells are said to provide a key role in "immune surveillance" against cancer. T-cells as mentioned above, secrete lymphokines, a diverse and potent array of biologically active molecules with a variety of effects. Some select examples of these T-cell lymphokines include the interleukin 2 (T-cell growth factor), B-cell growth factor, interferon (gamma), and macrophages produce lympholines (IL-1). These lymphokines serve at least two roles in the immune response, one is the regulation of immunity and the other is actual direct cytotoxicity (destruction) of tumor cells or virus-infected cells.

Immunomodulating agents activate or inhibit the process of lymphocyte proliferation. Normal lymphocyte proliferation is due to various interactions between antigens, macrophages, T- and B-lymphocytes. Additionally, certain B-lymphocytes can be activated by T-lymphocytes while others are independent of the T-lymphocytes and are activated only by antigens directly. Activated T-lymphocytes can cause macrophages to produce a molecule known as interleukin 2(IL-2) which in turn activates T-cells, which then stiumulate other T- and B-lymphocytes. Activated macrophages can produce a molecule known as interleukin 1 (IL-1) which further induces T-lymphocyte activation. Chemicals, called mitogens can trigger DNA synthesis and mitosis, which are signs of activity in T-and B-lymphocytes. Some mitogens affect only one type of lymphocyte while others affect many types. Immunomodulating agents of various kinds and in varying amounts affect the complex interactions between the components of the immune system. The compounds and compositions of this invention act as immune stimulators and affect both T- and B-lymphocytes.

The immune system has been linked to some aspects of aging and may be important in protecting against cancer. The system is necessary for the recognition of changing or aging cells, such as worn out red blood cells, and their subsequent destruction, and for this reason is vital to normal body functions. One theory in the case of cancer is that the transformation of cells to the malignant state may occur fairly frequently but these changed cells are recognized as "not self" and destroyed. Some carcinogens may work by depressing the immune response rather than by transforming cells themselves to a malignant state. This would mean that the body would no longer destroy the spontaneously transformed cells and a cancerous growth could escape, resulting in a tumor. Immunostimulation could be useful in treating such cancers.

Also, certain tumors which develop in man produce as a result of their growth an immunodepressed state in the host (i.e. leukemias, lymphomas, respiratory cancers, and HTLV induced tumors).

Some of the methods of treating cancer, surgery, cytotoxic chemotherapy, and radiation for example, can result in a suppression or drastic variation of the normal functions of the immune system. Immunostimulatory drugs, such as the compounds and compositions of this invention can be very effective in combating and/or preventing various infections which can result due to the depressed immune system.

## OBJECTS OF THE INVENTION

An object of the invention is to provide compounds having immunomodulatory activity, particularly immunostimulatory activity.

Another object of the invention is to provide methods for producing such compounds. some of which are novel.

Another object of the invention is to provide novel compositions effective in the treatment of immune disorders, especially those requiring stimulation of the immune system.

Other objects, advantages and novel features of the invention will become apparent from the following detailed description.

## DESCRIPTION OF THE INVENTION

It has been discovered that the acid catalyzed Michael reaction products of D- or L-ascorbic acid and certain selected ketones are useful as immunomodulators. More specifically the useful reaction products of the invention are those obtained by reactions of ascorbic acid with $\alpha,\beta$-unsaturated alicyclic ketones containing from 4 to 7 carbon atoms, or with vinyl aliphatic ketones in which the saturated moiety of the aliphatic moiety may be substituted with substituents such as alkyl containing up to four carbon atoms or similar haloalkyl groups especially fluoroalkyl groups such as trifluoromethyl.

Preferred compounds of the invention may be represented by the formulas:

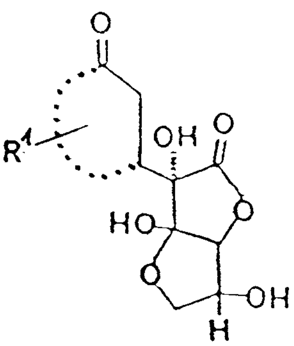

(I)

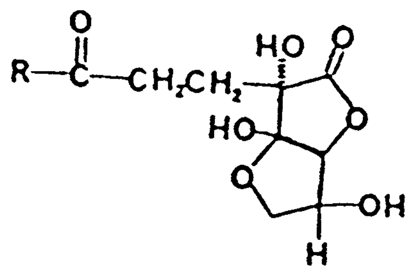

(II)

wherein $R^1$ is hydrogen, an alkyl group having up to 4 carbon atoms or a ($C_1$-$C_4$) haloalkyl group, especially a ($C_1$-$C_4$) fluoroalkyl group, with the proviso that $R^1$ cannot be either at the $\alpha$ or $\beta$ carbon atom, and the dotted line represents the $CH_2$ units which are necessary to complete the alicyclic ring to 4 to 7 carbon atoms and R represents an alkyl group containing from 1 to 5 carbon atoms.

Typical compounds within the scope of this invention include the reaction products of methyl vinyl ketone and L-ascorbic acid or 2-cyclohexenone and L-ascorbic acid. The Structures of these compounds may be represented by the following formulas:

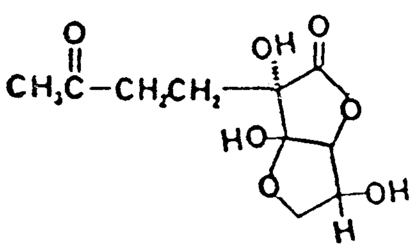

KBBL

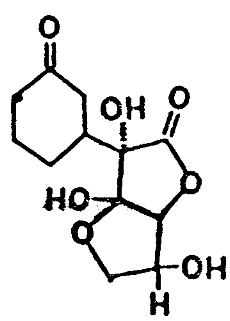

KCBL

The chemical names for these compounds are, respectively:

2-(3-ketobutyl)-2-hydroxy-3-keto-4-dihydroxyethyl butyrolactone <3,6>cyclohemiketal (KBBL) and

2-(3-ketocyclohexyl)-2-hydroxy-3-keto-4-dihydroxyethyl butyrolactone <3,6>cyclohemiketal (KCBL).

Presently preferred compounds within the scope of the invention are those in which the alicyclic ring of the ketone is unsubstituted and contains 5 or 6 carbon atoms and those in which R contains 1 or 2 carbon atoms. These are preferred because of their activity and because the starting compounds are readily and economically available. 3-Methyl-2-cyclohexenone does not react under the same conditions with ascorbic acid. 2-Cycloheptenone reacts, but very sluggishly.

The reaction is carried out in an aqueous medium at ambient temperatures in the presence of a catalytic amount of a strong acid, suitably a mineral acid such as sulfuric or a halogen acid, preferably hydrochloric acid. Preferably the reaction is conducted in an inert atmosphere such as nitrogen or helium.

The preferred reaction medium is water, although other solvents may be added, especially water miscible solvents such as lower alkanols, typically methanol, ethanol, or cyclic ethers such as tetrahydrofuran, or ketones particularly acetone. These will assist in dissolving some of the higher molecular weight, or hydrophobic reactants.

Reaction is effected at a temperature of from about 20°Cto 45°C for a period of from about 2 to 24 hours. The reaction period is not critical. It depends principally on the quantities of the reactants. The reaction is readily followed by conventional analytical methods to determine when the reaction is complete, or when continued reaction is not warranted by expected increase in yield. High performance liquid chromatography is a convenient tool.

Generally equimolar quantities of the reactants will be employed. However, in certain instances it may be desirable to use a molar excess, e.g., up to about a 10% molar excess of one of the reactants to assure as complete a reaction as possible.

As aforesaid, any of a variety of strong acids can be employed to catalyze the rection. Typically 0.1% to 1.5% by weight of acid based on the total weight of reactants will be employed. In an aqueous medium, hydrochloric acid is preferred since it is readily removed by precipitation as a chloride salt. However, a stronger carboxylic acid such as trichloroacetic acid or trifluoroacetic acid may be used.

It is surprising to find a Michael addition reaction catalyzed by acid. Usually this type of reaction which is an addition of an active methylene compound to an activated unsaturated system is catalyzed by base.

The compounds of this invention which are based on alicyclic ketones are novel. Certain of those based on aliphatic ketones have been described. For example, the reaction product of methyl vinyl ketone and ascorbic acid was described by Fodor et al in Tetrahedron Vol. 39, No. 13, pages 2137 to 2145 (1983). The reaction described in that publication is not an acid catalyzed reaction and is much less rapid than the reaction described herein. Additionally the reaction described in the publication is far too slow to be practical for use to prepare reaction products on a large scale based on cyclic ketones.

The ability of the compounds of this invention to stimulate an immune response has been established by a number of art recognized tests.

One of these tests is the lymphocyte blastogenesis assay which measures the ability of the compound under test to affect DNA synthesis and mitosis of T- and B- lymphocytes isolated from mouse spleens.

Mitogens are substances which stimulate DNA synthesis and mitosis. The mitogens used in these studies were phytohemagglutinin (PHA) which is isolated from the red kidney bean and concanavalin-A (Con-A) which is isolated from the jack bean. Con-A binds to specific receptors (glycoproteins) containing mannosyl or glycosyl moieties and stimulates all murine T-cells to synthesize DNA, divide, and release lymphokines. Con-A in a soluble form allows distinction between T- and B-cells in the mouse, because although both T- and B-cells can bind 10-6 molecules of Con-A per cell, only T-cells are stimulated when this lectin is presented in a soluble form. PHA stimulates only subpopulations, T-2 cells, of T-cells in the mouse. In humans, both T- and B-cells are probably stimulated. The activation of B-cells by PHA may be indirect and mediated by the release of soluble mediators from PHA-activated T-cells.

The lymphocyte blastogenesis test is a method to assess the ability of immunocompetent T- or B-cells to respond to a polyclonal mitogen (i.e. PHA or Con-A) or a specific antigen. It may be performed on lymphocytes obtained from mice treated with immunostimulators in vivo or the entire assay can be performed in vitro. The assay as described below uses minimal doses of polyclonal mitogens to induce blastogenesis (proliferation measured by DNA synthesis), in order to be able to assess the phenomena of amplification. Hence, the procedure is designed to test the ability of potential immunomodulators to restore normal immunologic parameters.

The lymphocyte blastogenesis test is carried out as follows:

1. Sacrifice two mice/experimental group by cervical dislocation.
2. Immerse mice in a mild disinfectant solution (Povadyne).
3. Remove spleens and place in a sterile 6 well plate containing 5 ml/well of RPMI-1640.
4. Make a single cell suspension by mincing spleens with a sterile toothed forcep.

5. Place cell suspension in a sterile centrifuge tube and allow large clumps to settle for 10 minutes.
6. Remove single cell suspension by pipetting supernatant into another sterile centrifuge tube.
7. Centrifuge cell suspension for 10 mins. at 1100 RPM in GLC-2B.
8. Aseptically remove supernatant and discard.
9. Resuspend cell button in 5 mls of RPMI-1640, and centrifuge. Wash cells in this manner two more times.
10. Resuspend cells in 5 mls of RPMI-1640 containing 10-15% Human AB heat-inactivated (Pel-Freeze, Rogers, AR or BioBee, Boston, MA).
11. Perform viable cell count using 0.25% trypan blue exclusion dye made in physiological isotonic saline. Non-viable cells stain blue.
12. Adjust viable cell concentration to 5.0 x 10-6 cells/ml in RPMI-1640 containing human AB sera.
13. Aliquot in sextuplicate wells of a 96 well sterile round bottom tissue culture plate with 0.1 ml/well of the various cell suspensions to be tested.
14. Add to above replicate sextuplicate cells 2.5, 5.0 or 7.5 ug/ml of Con A, and to replicate again of 10, 15 or 20 ug/ml of PHA.
15. Include in the experiment a control plate which contains the same cell groups as above, but receive a 0.1 ml of media instead of mitogen.
16. Humidify plate by filling outside wells of plate with media.
17. Incubate plates at 3 C. with 5% CO-2 for 48 hours.
18. After 48 hours all wells receive 0.025 mls of a 0.4 microcurie/ml solution of C-14 methyl thymidine and incubate at 37 C, 5% CO-2 for 18 hours.
19. The cells are harvested using a Brandel M-12 Cell Harvestor (Brandel, Rockfille, MD) onto filter paper discs using phosphate buffered saline at physiological osmolarity. (285-320 mos).
20. The filter paper disks are placed into Packard mini-scintillation vials and allowed to dry for 18 hours.
21. Once dried, the vials are filled with 2 mls of a scintillation cocktail containing 4 liters of scintillation grade toluene, 16.0 g of 2,5-diphenyloxazole (PPO) and 0.4 g of 1,4-Bis (2-(5-Phenyloxazolyl) benzene (POPOP).
22. The vials are counted in a LKB 1212 Rackbeta (LKB Instruments, Gaithersburg, MD) Liquid scintillation counter for two minutes/vial.

Table I and II show the results of the lymphocyte blastogenesis assay on two isomers of KCBL. It will be noted that both isomers gave statistically significant increases in counts per minute based upon C-14 thymidine incorporation of DNA.

TABLE 1

| EFFECT OF KCBL-A ON THE LYMPHOCYTE BLASTOGENESIS ASSAY | | | | | |
|---|---|---|---|---|---|
| DRUG DOSE mg/kg | RPMI CONTROL | PHA 14 ug/ml | % INCREASE | CON-A 6 ug/ml | % INCREASE |
| 0 | 103.0 +/- 14.0 | 1727.0 +/- 77.0 | -- | 3612.0 +/- 276.0 | -- |
| 12.5 | 94.0 +/- 18.0 | 1450.0 +/- 50.0 | -- | 3412.0 +/- 251.0 | -- |
| 25.0 | 77.0 +/- 12.0 | 2178.0* +/-139.0 | 26.1 | 5657.0* +/- 408.0 | 56.6 |
| 50.0 | 50.0 +/- 8.0 | 2369.0* +/-132.0 | 37.1 | 5059.0* +/- 408.0 | 40.0 |
| 100.0 | 76.0 +/- 9.0 | 2384.0* +/-170.0 | 38.0 | 4104.0 +/-1287.0 | 13.6 |

* Significant increase over the controls (p 0.05); +/- refers to standard deviation.

TABLE II

| Effect of KCBL-B ON THE LYMPHOCYTE BLASTOGENESIS ASSAY | | | | | |
|---|---|---|---|---|---|
| DRUG mg/kg | RMPI | PHA 14.0 ug/ml | % INCREASE | CON-A 2.25 ug/ml | % INCREASE |
| Mean 0 S.D. | 43.7 +/- 4.2 | 1545.8 +/- 153.3 | -- | 388.2 +/-102.1 | -- |
| 12.5 | 67.6 +/- 7.7 | 1733.1* +/- 100.0 | 12.1 | 865.6* +/-277.3 | 122.9 |
| 25 | 84.3 +/- 13.1 | 3512.1* +/- 779.3 | 127.3 | 1309.6* +/-230.3 | 237.4 |
| 50 | 56.2 +/- 7.5 | 2540.7* +/- 121.7 | 64.4 | 1130.6* +/-121.7 | 191.2 |
| 100 | 84.9 +/- 9.9 | 3515.4* +/- 151.9 | 127.5 | 1736.7* +/-261.1 | 347.4 |
| 200 | 52.0 +/- 9.8 | 3305.8* +/- 111.0 | 114.1 | 2008.0* +/-458.1 | 417.5 |

* Indicates significant increase over controls (p 0.05); +/- refers to standard deviation.

RPMI is a semi-synthetic cell growth medium available from (Hazelton Labs, Inc. Denver, PA) and comprises all the essential amino acids, vitamins, buffers, and salts required for mammalian cell growth except serum growth factors supplied using an animal (bovine) source.

Table III records the results of the lymphocyte blastogenesis assay with KBBL which is the Michael addition product of L-ascorbic acid and methyl vinyl ketone. As will be observed the compound is stimulatory at all the doses of PHA and Con-A tested as well as at all doses of the drug used to treat the mice prior to the assay.

TABLE III

| EFFECT OF KBBL ON THE LYMPHOCYTE BLASTOGENESIS ASSAY | | | | | | | | | |
|---|---|---|---|---|---|---|---|---|---|
| DRUG DRUG mg/kg | RPMI CONTROL | PHA 10.5ug | % INCR. | PHA 14ug | % INCR. | CON-A 1.5ug | % INCR. | CON-A 2.25ug | % INCR. |
| 0 | 26<br>5 | 1739<br>113 | -- | 2323<br>44 | -- | 468<br>194 | -- | 1508<br>414 | -- |
| 25 | 255<br>83 | 7935*<br>667 | 356 | 10994*<br>152 | 373 | 3572*<br>526 | 663 | 5398*<br>449 | 260 |
| 50 | 92<br>24 | 4579*<br>776 | 163 | 7295*<br>477 | 214 | 2539*<br>436 | 443 | 5802*<br>1585 | 285 |
| 100 | 33<br>6 | 3540*<br>768 | 103 | 4878*<br>428 | 110 | 1051*<br>344 | 54 | 3056<br>937 | 103 |
| 200 | 55<br>60 | 47-7*<br>655 | 171 | 6963*<br>621 | 200 | 2571*<br>865 | 449 | 6891*<br>1026 | 357 |

* Indicates statistically significant at $p < 0.05$; +/- refers to standard deviation.

The Jerne hemolytic plaque assay is an assay procedure which measure the IgM or IgG isotoypes of specific antibody produced by antigen used to immunize the mice. The procedure demonstrates antibody production to T-cell dependent antigens by single B-lymphocytes. The direct assay detects IgM and the indirect assay detects IgG-specific antibody.

The procedure is as follows:

Washed spleen cells are added to an agarose (FMC, Rockland, ME) preparation containing sheep red blood cells (SRBC) and guinea pig complement. A drop of this mixture is transferred to a small petri dish; a cover slip is then placed over the drop to flatten it. The agarose prep is allowed to solidify. Then, the dishes are placed in a small humidified chamber inside a CO-2 incubator overnight.

Small pinpoint cleared areas (plaques) will be observed under the cover slip. The cleared areas are caused by antibody, specific to the SRBC's being released by stimulated spleen cells and in conjunction with the complement, lyse surrounding SRBC's. These plaque forming cells (PFC's) are counted, and calculations are performed to obtain PFC's per 1 x 10-6 spleen cells.

| Reagents: | Sheep Red Blood Cells Sea Plaque Agarose RPMI-1640 | Guinea Pig Complement 30 mm Petri Dishes 22 x 22 x 1 1/2 cover slips |
|---|---|---|

Methods:

1. Spleens are harvested from mice according to a standard procedure and adjusted to a final concentration of 20% in RPMI-1640.
2. SRBC's are adjusted to a final concentration of 20% in RPMI-1640.
3. Reconstitute Guinea Pig Complement (GPC) (Hazelton-Dutchland Inc., Denver, PA) by adding buffered duluent directly to lyophilized GPC. Dilute 1:7 with RPMI-1640 by adding 3 ml of RPMI media to 0.5 ml complement.
4. Prepare Sea Plaque Agarose (FMC, Rockland, ME) at 0.7% to 25 ml of RPMI-1640, add 0.175 g of the agarose in a 50 ml sterile flask. Place over medium heat, use stirring bar at lowest speed to stir gently. Remove from heat prior to boiling. Place in 37 C water bath immediately.
5. Place all reagents (SRBC's, GPC and spleen cell preps) in water bath for 15 minutes to equilibrate to 37 C.
6. For each preparation to be tested, place a 12 x 75 mm glass test tube into rack in 37 C water bath. Add 0.7 ml of the agarose to each 12 x 75 tube.
7. Adjust three separate Rannin pipetman (Gilson, Middleton, WI) to 0.05, 0.1, 0.2 ml.
8. To the first 12 x 75 mm tubes containing agarose, add 0.2 ml of the first spleen cell prep. Next, add 0.05 ml of the complement. Add 0.05 ml of SRBC's. Mix very well, until SRBC's are in a uniform suspension.
9. Label the sides of both top and bottom of a petri dish to correspond to the cell group.
10. Using th 0.1 ml pipette, withdraw 0.1 ml of th agarose/cell prep and dispense into center of petri dish-top. Repeat immediately for bottom of petri dish.
11. Carefully place/drop a 22 x 22 mm cover slip squarely over the droplet. Do not move dishes until agarose settles and solidifies.
12. Repeat steps 6-11 for each of the remaining cell preps.
13. After agarose has solidified, transfer to small plastic humidified chamber. Place in incubator. Allow to incubate overnight at 37 C. Read results the following morning.
14. Count and record the number of hemolytic plaques per plate. Average plaque counts between the duplicate sets. Multiply the average number of plaques per group by 2.5 to obtain the number of plaque forming cells per million spleen cells.

Tables IV and V record the results of the Jerne assay on the two isomers of KCBL (A and B) and on KBBL respectively. All three drugs manifested a dose dependent increase compared to the control.

TABLE IV

EFFECT OF KCBL A AND B ON THE JERNE ASSAY

| | KCBL-A | | KCBL-B | |
|---|---|---|---|---|
| DRUG TREATMENT | PFC/1 MILLION CELLS | % INCREASE | PFC/1 MILLION CELLS | % INCREASE |
| VEHICLE CONTROL | -- | -- | 3.75 | -- |
| **SRBC CONTROL** | **56.25** | -- | **47.50** | -- |
| 200 mg/kg | 177.50 | 215 | 128.75 | 171 |
| 100 mg/kg | 235.00 | 318 | 157.5 | 232 |
| 50 mg/kg | 165.00 | 193 | 151.25 | 218 |
| 25 mg/kg | 115.00 | 104 | 66.25 | 39 |
| 12.5 mg/ml | 112.00 | 100 | -- | -- |

TABLE V

| EFFECT OF KBBL ON THE JERNE ASSAY | | |
|---|---|---|
| TREATMENT GROUP | PFC/1 MILLION CELLS | % INCREASE |
| VEHICLE CONTROL | 0.5 | -- |
| SRBC CONTROL | 23.5 | -- |
| KBBL 200 mg/kg | 48.5 | 106.4 |
| KBBL 100 mg/ | 48.0 | 104.3 |
| KBBL 50 mgkg | 36.0 | 70.2 |

The biologically active compounds of this invention may be administered alone or in conbination with acceptable pharmaceutical carriers, the choice of which is determined by the preferred route of administration, the solubility of the compound and standard pharmaceutical practice. For oral administration, the compounds may be administered in the form of tablets containing excipients such as buffers, starch or milk sugar. Aqueous solutions and elixirs which may be buffered and sweetened or flavored may also be used. For intra-articular injection aqueous suspensions may be employed. In this case various suspending and wetting agents may be added to the composition to obtain a suspension not tending to settle out easily or to pack down in the bottle in which it is stored. Intramuscular and subcutaneous dosage forms may also be prepared by standard pharmaceutical practice.

The compounds may be used in association with other therapeutic agents including, for example, antibiotics or antiviral agents. It may also be useful to employ the synthetic immunostimulators in association with natural immunostimulators such as interleukin 1 and 2, or interferon or it's synthetic inducers (i.e. poly IC-LC etc.), B-cell growth factors, or tumor necrosis factor. They may be administered by any of the usual routes of administration intramuscular or intraveneous.

The physician or veterinarian in attendance will determine the optimum dosage in consideration of such factors as age, weight and general health of the subject. A dose which will be effective to stimulate an immunomodulatory response will normally be from about 100 to 50 mg/kg body weight, although wide variations are possible. The dosage may be administered in one treatment or in several treatments given over a period of time.

The compositions of the invention may be made available in dosage unit forms typically containing from 2 to 1 of active ingredient per dosage unit.

EXAMPLE 1

2-(3-Ketobutyl)-2-Hydroxy-3-Keto-4-Dihydroxyethyl Butyrolactone 3,6 Cyclohemiketal (KBBL)

L-ascorbic acid (22.0 g, 0.125 mole) was added to 88 ml water that had been degassed for 1 hour with nitrogen. Methyl vinylketone (8.75 g, 0.125 mole) was added dropwise to the resulting solution, followed by 0.3ml concentrated hydrochloric acid. The reaction mixture was allowed to stir at ambient temperature for 24 hours until HPLC analysis showed the complete consumption of methyl vinyl ketone. The HCl catalyst was removed by the addition of 2.0 g silver carbonate. The filtrate, after centrifugation, was frozen and freeze-dried to give 28.80 g (94%) of crude product. The solid was dissolved in 550 ml boiling ethyl acetate, which upon cooling gave 13.74 g of white crystalline solid. Concentration of the filtrate gave 3.35 g of the second crop of crystalline solid. Both crops were combined and recrystallized in 450 ml hot ethyl acetate to give pure produce (13.30 g, 43.3%), ml 124-135, [2]D-25 = +23.1 (C = 1.02, methanol).

The corresponding ethyl vinylketone product was similarly prepared. Its melting point was 65-67.

EXAMPLE 2

Preparation Of The Two Diastereoisomers Isomers Of KCBL

Distilled water (704 mL) was purged for 1 hour with nitrogen and L-ascorbic acid (Mallinckrodt USP Grade) (176.0 g, 1.0 mole) was added. To the resulting solution 2-cyclohexenone (Aldrich Chemical) (96.0 g, 1.0 mole) was added dropwise with stirring. One half hour after the addition of the 2-cyclohexenone, concentrated hydrochloric acid (2.4 mL) was added. The solution was allowed to stand undisturbed at room temperature for 24 hours when the formation of crystalline solid was observed. The mixture was cooled in ice-water bath and filtered by suction to give 88.95 g (33%) of a crude first crop. The filtrate was allowed to cool in a refrigerator overnight whereupon more solid precipitated. The solid was filtered by suction to give 92.72 g (34%) of a crude second crop. High pressure liquid chromatography (HPLC) analysis (MCH 10 column with 80% water/methanol as eluent and 1.0 mL/min flow rate) indicated that the crude first crop consisted of approximately 90% isomer 1 (retention time 4.5 min) and the crude second crop consisted of approximately 55% isomer 2 (retention time 4.2 min).

The crude first crop was recrystallized in hot water (180 mL) yielding 58.90 g (22%) of pure (by HPLC) isomer 1, mp 155-156°, $[\alpha]^{19}$ = +7.1° (C = 2.06, methanol), $[\alpha]^{19}$ = -2.7° (C = 1.0, acetone).

The crude second crop was recrystallized three times in hot water (147 mL, 140 mL and 100 mL) Yielding 28.75 g (11%) of pure (by HPLC) isomer 2, mp. 170-171° , , $[\alpha]^{22}$ = +24.2° (C = 2.0, methanol), $[\alpha]^{22}$ = 6.5 (C = 0.8, acetone)

Example 3

Tablet Formulation

| Formula: | Mg/tablet |
|---|---|
| KBBL | 200.00 |
| Citric acid | 1.00 |
| Lactose | 33.00 |
| Dicalcium phosphate | 70.00 |
| Pluronic, F-68 | 30.0 |
| Sodium Lauryl Sulfate | 15.00 |
| Polyvinylpyrrolidone | 15.00 |
| Carbowax 1500 | 5.00 |
| 3A alcohol 50 ml./1000 tablets | |
| Corn Starch | 30.00 |
| Dry: | |
| Sodium Lauryl Sulfate | 3.00 |
| Magnesium stearate | 3.00 |
| Total weight | 350.00 |

Procedure. -- Mix together the KBBL, citric acid, Pluronic F-68, sodium lauryl sulfate, lactose and dicalcium phosphate. Screen through No. 60 mesh screen. Granulate the screened mix with an alcoholic solution containing the polyvinylpyrrolidone, Carbowax 1500 and 6000. Add additional alcohol, if necessary, to bring powder mix to a pasty mass. Add corn starch and continue mixing until uniform damp granules are formed. Pass the damp granulation through a No. 10 screen and dry in an oven at 100° C for 12-14 hours. Screen the dried granulation using a No. 16 screen, add sodium lauryl sulfate and magnesium stearate, mix and compress on a tablet machine to specifications.

Example 4

| Capsule Formulation | |
|---|---|
| Formula: | Mg./capsule |
| KCBL Isomer 1 | 100.00 |
| Citric acid | 1.00 |
| Pluronic F-68 | 40.0 |
| Sodium lauryl sulfate | 20.00 |
| Lactose | 238.00 |
| Magnesium stearate | 1.00 |

Procedure. -- Mix together the KCBL, citric acid, Pluronic F-68, sodium lauryl sulfate and lactose. Pass through a No. 80 screen. Add the magnesium stearate, mix and encapsulate into the proper size 2-piece gelatin capsule.

Example 5

| Parenteral Formulation | |
|---|---|
| Formula: | |
| KCBL Isomer 2 | mg/10 ml 200 |
| Benzyl alcohol, UF | mg/10 ml 50.0 |
| Methyl paraben, USP | mg/10 ml 18.0 |
| Propyl paraben, USP | mg/10 ml 2.0 |
| Water | ml 10 |

Procedure. -- Dissolve the parabens in approximately 8.5 ml of water at 60 to 70° C. cool the solution to 40 °C and add the benzyl alcohol. Cool the resultant solution to room temperature and add the KCBL. Place the suspension in a sterile receptacle. Fill suitably sized vials cap loosely and autoclave for one-half hour at 110 C (15 p.s.i.g.). Each milliliter of this formulation delivers 20 mgs. of active compound.

**Claims**

**1.** A compound of formula I

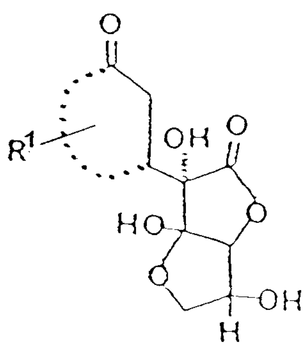

(I)

wherein $R^1$ is hydrogen, an alkyl group having up to 4 carbon atoms or a ($C_1$-$C_4$) haloalkyl group, with the proviso that $R^1$ cannot be either at the $\alpha$ or $\beta$ carbon atom, and the dotted line represents the $CH_2$ units which are necessary to complete the alicyclic ring to 4 to 7 carbon atoms and the ascorbic acid residue may have either D-or L-configuration.

**2.** A compound of claim 1 in which the alicyclic ketone contains 5 or 6 carbon atoms.

**3.** A compound of claims 1 or 2 in which the ($C_1$-$C_4$) haloalkyl group is a ($C_1$-$C_4$) fluoroalkyl group.

4. A compound of any of claims 1 to 3 in which the ascorbic acid is L-ascorbic acid.

5. A pharmaceutical composition containing a pharmaceutically acceptable carrier together with a compound according to claim 1
or together with a compound of formula II

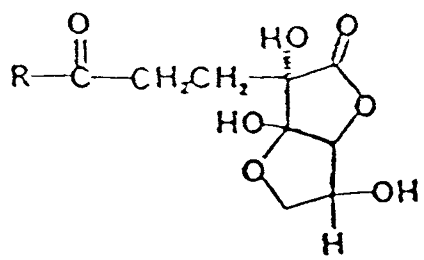

(II)

wherein R represents an alkyl group containing from 1 to 5 carbon atoms and the ascorbic acid residue may have either D- or L-configuration.

6. A composition of claim 5 in which the alicyclic ketone contains 5 or 6 carbon atoms.

7. A composition of one or both of claims 5 to 6 in which the ascorbic acid is L-ascorbic acid.

8. A composition of claim 5 in which R contains 1 or 2 carbon atoms.

**Patentansprüche**

1. Verbindung der Formel I

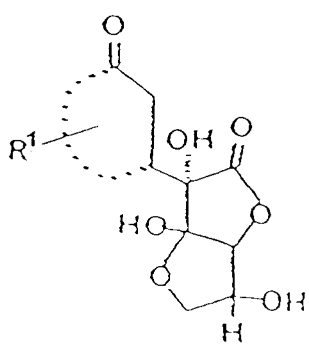

(I)

worin $R^1$ = Wasserstoff, eine Alkylgruppe mit bis zu 4 Kohlenstoffatomen oder eine $(C_1\text{-}C_4)$-Halogenalkylgruppe ist, unter der Voraussetzung, daß $R^1$ weder am $\alpha$-noch am $\beta$-Kohlenstoffatom sein kann und daß die gepunktete Linie die $CH_2$-Einheiten darstellt, die notwendig sind, um den alicyclischen Ring auf 4 bis 7 Kohlenstoffatome zu vervollständigen und der Ascorbinsäurerest entweder D- oder L-Struktur hat.

2. Verbindung nach Anspruch 1, worin das alicyclische Keton 5 oder 6 Kohlenstoffatome enthält.

3. Verbindung nach Anspruch 1 oder 2, worin die $(C_1\text{-}C_4)$-Halogenalkylgruppe eine $(C_1\text{-}C_4)$-Fluoralkylgruppe ist.

4. Verbindung nach einem der Ansprüche 1 bis 3, worin die Ascorbinsäure L-Ascorbinsäure ist.

5. Pharmazeutisce Zusammensetzung, die einen pharmazeutisch geeigneten Träger zusammen mit einer Verbindung nach Anspruch 1 enthält
oder zusammen mit einer Verbindung der Formel II

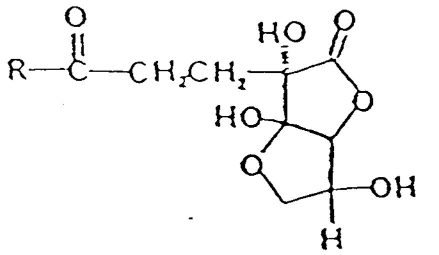

(II)

worin R eine Alkylgruppe mit 1 bis 5 Kohlenstoffatomen bedeutet und der Ascorbinsäurerest entweder D- oder L-Struktur hat.

6. Zusammensetzung nach Anspruch 5, worin das alicyclische Keton 5 oder 6 Kohlenstoffatome enthält.

7. Zusammensetzung nach einem oder beiden der Ansprüche 5 bis 6, worin die Ascorbinsäure L-Ascorbinsäure ist.

8. Zusammensetzung nach Anspruch 5, worin R 1 oder 2 Kohlenstoffatome enthält.

**Revendications**

1. Composé de formule I :

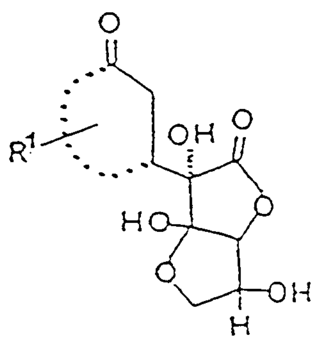

(I)

dans laquelle $R^1$ est de l'hydrogène, un groupe alkylé ayant jusqu'à 4 atomes de carbone ou un groupe haloalkylé ($C_1$-$C_4$), à condition que $R^1$ ne soit pas sur l'atome de carbone $\alpha$ ou $\beta$, et la ligne interrompue représente les unités $CH_2$ qui sont nécessaires pour compléter le noyau alicyclique à 4 à 7 atomes de carbone, et le résidu d'acide ascorbique peut avoir soit la configuration D, soit la configuration L.

2. Composé selon la revendication 1, dans lequel la cétone alicyclique contient 5 ou 6 atomes de carbone.

3. Composé selon la revendication 1 ou 2, dans lequel le groupe haloalkylé ($C_1$-$C_4$) est un groupe fluoroalkylé ($C_1$-$C_4$).

4. Composé selon l'une quelconque des revendications 1 à 3, dans lequel l'acide ascorbique est l'acide ascorbique L.

5. Composition pharmaceutique contenant un support pharmaceutiquement acceptable en même temps qu'un composé selon la revendication 1, ou en même temps qu'un composé de formule II :

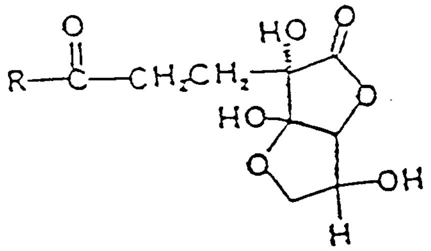

(II)

dans laquelle R représente un groupe alkylé contenant de 1 à 5 atomes de carbone et le résidu d'acide ascorbique peut avoir soit la configuration D, soit la configuration L.

**6.** composition selon la revendication 5, dans laquelle la cétone alicyclique contient 5 ou 6 atomes de carbone.

**7.** Composition selon l'une ou les deux revendications 5 et 6, dans laquelle l'acide ascorbique est l'acide ascorbique L.

**8.** Composition selon la revendication 5, dans laquelle R contient 1 ou 2 atomes de carbone.